# EUROPEAN PATENT APPLICATION

(11) **EP 1 535 630 A1**
(43) Date of publication of application: **01.06.2005**
(21) Application number: 03765368.0
(22) Date of filing: 23.07.2003
(51) Int. Cl.: A61K 45/00, A61K 31/4985, A61K 31/517, A61K 31/675, A61P 3/10, A61P 9/10, A61P 9/12, A61P 43/00, C07D 239/94, C07D 417/12, C07D 471/04

(54) **PREVENTIVE FOR THE ONSET OF DIABETES**

(30) Priority: 23.07.2002 JP 2002213898; 18.12.2002 JP 2002366844
(71) Applicant: Sankyo Company, Limited, Tokyo 103-8426 (JP)
(72) Inventor: YOSHIDA, Taishi, SANKYO COMPANY, LIMITED, Tokyo 140-8710 (JP); OKUNO, Akira, SANKYO COMPANY, LIMITED, Tokyo 140-8710 (JP)
(74) Representative: Gibson, Christian John Robert
(86) International application number: PCT/JP2003/009348
(87) International publication number: WO 2004/009118

(57) **Abstract**

The present invention relates to a pharmaceutical composition containing an FBPase inhibitor as an active ingredient, which is a preventative agent for diabetes mellitus, diabetic complications, hyperlipidemia, hypertension, arteriosclerosis, macroangiopathy, and the like and a therapeutic agent for IGT, and which can safely and easily be administered for a long period.

## Description

### [Technical field]

The present invention relates to preventive agents containing fructose 1,6-bisphosphatase (FBPase) inhibitors for diabetes mellitus, diabetic complications, hyperlipidemia, hypertension, arteriosclerosis, macroangiopathy, and so forth (preferably preventive agents for diabetes mellitus).

Further, the present invention relates to therapeutic or preventive agents containing FBPase inhibitors for impaired glucose tolerance (IGT).

Moreover, the present invention relates to pharmaceutical compositions containing the FBPase inhibitors for the prevention of the diseases described above, to the use of the FBPase inhibitors in the manufacture of a medicament for the prevention or treatment of the diseases described above, to methods for the prevention or treatment of the diseases described above by administering a pharmacologically effective amount of the FBPase inhibitors to warm-blooded animals (preferably humans), or to the administration of the FBPase inhibitors for the prevention or treatment of the diseases described above.

### [Background art]

The number of patients with diabetes mellitus was said to be approximately 6.9 million in Japan and approximately 150 million worldwide in 1999, and the number of the patients is increasing each year. Thus it has been an important target to develop therapeutic methods for diabetes mellitus and related disorders.

Several drug therapies, such as insulin therapy, have been used for diabetes mellitus. However, the direct or indirect costs including the medical expenses for the therapy of the disease are over 1 × 10¹³ yen in the United States. Thus the large expense for the therapy of diabetes mellitus is now a new social problem.

According to The Diabetes Prevention Program in the United States, which was conducted on a large scale, the importance of preventative methods for diabetes mellitus was pointed out, rather than therapy of the disease, because the former requires lower cost than the latter. In fact, the results of the clinical trials indicated that either intensive lifestyle modulation therapy including moderate intensity of physical activity (exercise) or treatment with metformin was effective to prevent diabetes mellitus. Nevertheless, gastrointestinal symptoms such as diarrhea occurred in approximately 80 % of the patients treated with metformin [see, for example, The New England Journal of Medicine, 346, 393-403 (2002)]. Although it is rare, another adverse event of metformin, lactic acidosis, has been reported and it is also known that metformin is difficult to administer to patients with hepatic or renal disorders. Further, it is mentally difficult and time consuming to continue exercise. Hence novel preventative methods which are safe and can be continued for a long period have been looked for.

On the other hand, from the viewpoint of prevention of diabetes mellitus, various risk factors of the disease have been investigated. Out of them, IGT is known to be a disease associated with postcibal higher blood glucose level than that in healthy subjects and results in development to diabetes mellitus in the future at a high rate. More than that, according to recent studies, IGT associated with hyperinsulinemia is related to initial pathological changes observed in patients with arteriosclerosis, such as hypertrophy of the medial complex of the carotid arteries, ischemia―induced cardiographic changes and vasospastic angina pectoris. Thus the therapy for IGT itself is getting more important, in addition to the prevention of progression to diabetes mellitus from IGT [see, for example, Diabetologia, 38, 585-591 (1995)]. Further, for example, the study of the relationship between IGT and cardiovascular diseases is in progress [see, for example, Diabetes Care, 22, 920-924 (1999)].

Now, some compounds are known as FBPase inhibitors. However, it is not yet known that FBPase inhibitors are useful as preventive agents for diabetes mellitus, and preventive or therapeutic agents for IGT [see, for example, International publication number WO 01/47935, International publication number WO 99/47549 and Bioorganic & Medicinal Chemistry Letters, 11, 17-21 (2001)].

### [Disclosure of the invention]

Diabetes mellitus is a chronic metabolic disease with a major symptom of high blood glucose level. It is a risk factor to cause many diseases, for example, peripheral neuropathy, sympathetic nerve disorders, diabetic retinopathy, cataracts, blindness, diabetic nephropathy, renal disorder, cerebral infarction, myocardial infarction, angina pectoris, heart diseases, diabetic necrosis and infections. Further, hyperlipidemia is a symptom observed frequently in patients with diabetes mellitus and it is strongly suggested that hyperlipidemia is associated with diabetes mellitus. Hyperlipidemia is an important risk factor of macroangiopathy such as cerebral infarction, cerebral hemorrhage, angina pectoris, myocardial infarction and obstructive arteriosclerosis. Thus it is important to prevent diabetes mellitus, because prevention of diabetes mellitus links to avoidance of diabetic complications such as neuropathy, retinopathy, cataracts, microvessel disorders, arteriosclerosis and renal disorders, as well as protection from macroangiopathy, in which hyperlipidemia is one of the risk factors.

As the result of earnest investigations on the preventative agents for diabetes mellitus, which have great safety and can be used easily for a long period, the present inventors have found that certain agents comprising FBPase inhibitors exert excellent preventative effect for diabetes mellitus with great safety, and they completed their invention.

Further, the present inventors also discovered that the agents comprising FBPase inhibitors improved significantly IGT (particularly, IGT which is one of the preceding states of crisis of diabetes mellitus), and completed their invention.

Thus, the present invention provides pharmaceutical agents containing FBPase inhibitors, as preventative agents, which have great safety and can be used easily for a long period, for diabetes mellitus, diabetic complications, hyperlipidemia, hypertension, arteriosclerosis, macroangiopathy and so forth.

The present invention also provides pharmaceutical agents containing FBPase inhibitors, as therapeutic or preventive agents for IGT.

In the present invention, there is no particular limitation on the "FBPase inhibitor" provided that the agent inhibits FBPase activity. Such inhibitors may preferably include:
a compound of general formula (I) or a pharmacologically acceptable salt thereof: 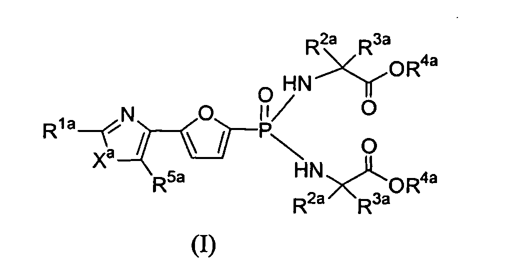 wherein X^{a} represents a nitrogen atom, an oxygen atom or a sulfur atom; R^{1a} represents a hydrogen atom, a halogen atom, a C₁₋₆ alkyl group, or an amino group (said amino group may optionally be substituted with one or two C₁₋₆ alkyl groups); R^{2a} and R^{3a} are the same or different, and each represents a hydrogen atom or a C₁₋₄ alkyl group; R^{4a} represents a C₁₋₄ alkyl group; and R^{5a} represents a hydrogen atom, a C₁₋₆ alkyl group or a C₁₋₆ alkylthio group;
a compound of general formula (II) or a pharmacologically acceptable salt thereof: 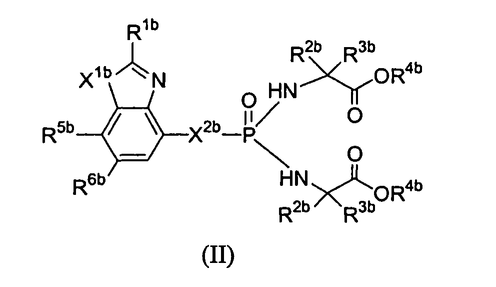 wherein X^{1b} represents an oxygen atom or a sulfur atom; X^{2b} represents a C₁₋₄ alkylene group, a C₁₋₄ oxyalkylene group (with the proviso that the phosphorous atom is attached to a carbon atom) or a C₁₋₄ thioalkylene group (with the proviso that the phosphorous atom is attached to a carbon atom); R^{1b} represents a hydrogen atom, a halogen atom, a C₁₋₆ alkyl group or an amino group (said amino group may optionally be substituted with one or more C₁₋₆ alkyl groups); R^{2b} and R^{3b} are the same or different, and each represents a hydrogen atom or a C₁₋₄ alkyl group; R^{4b} represents a C₁₋₄ alkyl group; and R^{5b} and R^{6b} are the same or different, and each represents a hydrogen atom, a halogen atom, a C₁₋₆ alkyl group, a C₁₋₆ alkoxy group or an amino group (said amino group may optionally be substituted with one or more C₁₋₆ alkyl groups);
a compound of general formula (III') or a pharmacologically acceptable salt thereof: 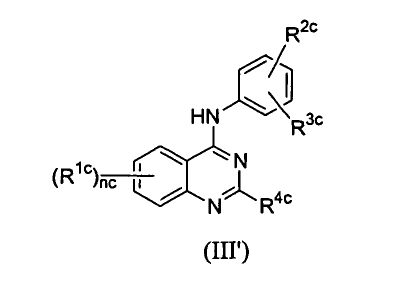 wherein R^{1c} represents a C₁₋₄ alkoxy group; R^{2c} and R^{3c} are the same or different, and each represents a thiazolyl group (said thiazolyl group may optionally be substituted with one methyl group or one methoxy group), an ethynyl group, a hydrogen atom, or a halogen atom; R^{4c} represents a C₁₋₃ alkyl group (said alkyl group may optionally be substituted with one imidazolyl group), a C₁₋₃ haloalkyl group, a C₁₋₃ aminoalkyl group or a hydrogen atom; and n^{c} represents an integer of from 1 to 3;
a compound of general formula (III) or a pharmacologically acceptable salt thereof: 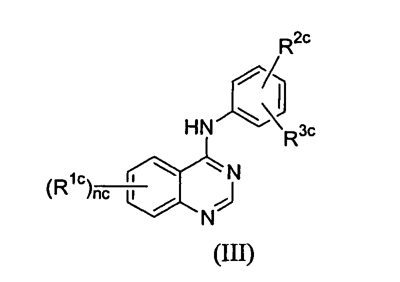 wherein R^{1c} represents a C₁₋₄ alkoxy group; R^{2c} and R^{3c} are the same or different, and each represents an ethynyl group, a hydrogen atom or a halogen atom; and n^{c} represents an integer of from 1 to 3; or
2-(4-cyanophenyl)-2-[(3S,11aS)-3-cyclohexylmethyl-8-hydroxy-1,4-dioxo-1,2,3,4,6,11,11a-octahydropyrazino[1,2-b]isoquinolin-2-yl]-N-[2-(4-hydroxyphenyl)ethyl]acetamide of the following formula (IV) or a pharmacologically acceptable salt thereof.

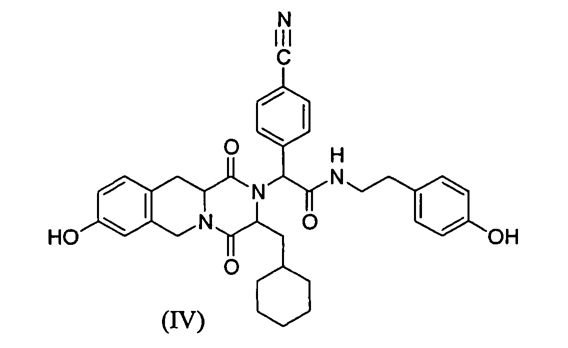

More preferred inhibitor is:
a compound of general formula (Ia) or a pharmacologically acceptable salt thereof: 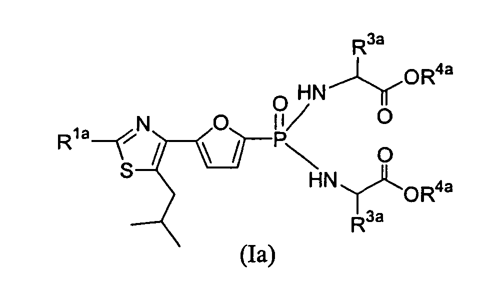 wherein R^{1a} represents an amino group (said amino group may optionally be substituted with one or more C₁₋₆ alkyl groups); R^{3a} represents a hydrogen atom or a C₁₋₄ alkyl group; and R^{4a} represents a C₁₋₄ alkyl group;
2-amino-5-isobutyl-4-{2-[5-(N,N'-bis((S)-1-ethoxycarbonyl)ethyl)phosphonamido]furanyl}thiazole,
2-amino-5-isobutyl-4- {2-[5-(O-(2-bis(N-(1-methyl-1-ethoxycarbonyl)ethyl)phosphonamido)furanyl)thiazole,
2-amino-5-propylthio-4-{2-[5-(N,N'-(1-(S)ethoxycarbonyl)ethyl)phosphonamido]furanyl}thiazole,
2-amino-5-propylthio-4-{2-[5-(N,N'-(1-methyl-ethoxycarbonyl)ethyl)phosphonamido]furanyl}hiazole, pharmacologically acceptable salts thereof or
a compound of general formula (IIIa') or a pharmacologically acceptable salt thereof: 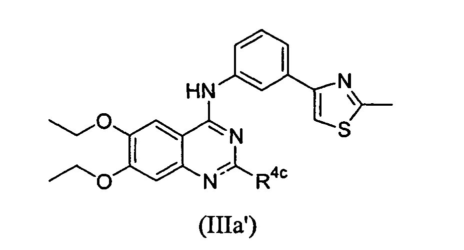
wherein R^{4c} represents a C₁₋₃ alkyl group (said alkyl group may optionally be substituted with one imidazolyl group), a C₁₋₃ haloalkyl group, a C₁₋₃ aminoalkyl group or a hydrogen atom.

Particularly preferred inhibitor is:
2-amino-5-isobutyl-4-{2-[5-(N,N'-bis((S)-1-ethoxycarbonyl)ethyl)phosphonamido]furanyl} thiazole,
2-amino-5-isobutyl-4- {2-[5-(O-(2-bis(N-(1-methyl-1-ethoxycarbonyl)ethyl)phosphonamido)furanyl)thiazole,
2-amino-5-propylthio-4- {2-[5-(N,N'-(1-(S)ethoxycarbonyl)ethyl)phosphonamido]furanyl} thiazole,
2-amino-5-propylthio-4- {2-[5-(N,N'-(1-methyl-ethoxycarbonyl)ethyl)phosphonamido]furanyl}thiazole,
(6,7-dimethoxy-quinazolin-4-yl)-(3-ethynyl-4-fluoro-phenyl)amine,
(6,7-diethoxy-quinazolin-4-yl)-[3-(2-methyl-thiazol-4-yl)phenyl]amine,
(2-aminoethyl-6,7-diethoxy-quinazolin-4-yl)-[3-(2-methyl-thiazol-4-yl)phenyl]amine, or pharmacologically acceptable salts thereof.

The compounds of general formulae (I), (Ia), (II) and 2-amino-5-isobutyl-4-{2-[5-(N,N'-bis((S)-1-ethoxycarbonyl)ethyl)phosphonamido]furanyl}thiazole, 2-amino-5-isobutyl-4- {2-[5-(O-(2-bis(N-(1-methyl-1-ethoxycarbonyl)ethyl)phosphonamido)furanyl} thiazole, 2-amino-5-propylthio-4- {2-[5-(N,N'-(1-(S)ethoxycarbonyl)ethyl)phosphonamido]furanyl}thiazole, and 2-amino-5-propylthio-4-{2-[5-(N,N'-(1-methyl-ethoxycarbonyl)ethyl)phosphonamido]furanyl}thiazole, and pharmacologically acceptable salts thereof can be prepared according to those methods disclosed in International publication number WO 01/47935.

The compound of general formula (III'), the compound of formula (IIIa'), the compound of formula (IIIb), and the compound of formula (IIIc), and pharmacologically acceptable salts thereof are disclosed in Journal of Medicinal Chemistry, 45, 3865-3877 (2002) and these compounds can be prepared in a procedure similar to that described above.

The compound of general formula (III), (6,7-dimethoxy-quinazolin-4-yl)-(3-ethynyl-4-fluoro-phenyl)amine of formula (IIIa) and pharmacologically acceptable salts thereof can be prepared according to those methods disclosed in Bioorganic & Medicinal Chemistry Letters, 11, 17-21 (2001) and International publication number WO 01/47935.

2-(4-Cyanophenyl)-2-[(3S,11aS)-3-cyclohexylmethyl-8-hydroxy-1,4-dioxo-1,2,3,4,6,11,11a-octahydropyrazino[1,2-b]isoquinolin-2-yl]-N-[2-(4-hydroxyphenyl)ethyl]acetamide of formula (IV) and a pharmacologically acceptable salt thereof can be prepared according to those methods disclosed in International publication number WO 99/47549.

In addition, 2-amino-5-isobutyl-4-{2-[5-(N,N'-bis((S)-1-ethoxycarbonyl)ethyl)phosphonamido]furanyl}thiazole can be described as L-alanine, N,N'-[[5-[2-amino-5-(2-methylpropyl)-4-thiazolyl]-2-furanyl]phosphinylidene]bis-, diethylester. 2-Amino-5-propylthio-4-{2-[5-(N,N'-(1-(S)ethoxycarbonyl)ethyl)phosphonamido]furanyl}thiazole can be described as L-alanine, N,N'-[[5-[2-amino-5-propylsulfanyl-4-thiazolyl]-2-furanyl]phosphinylidene]bis-, diethylester.

In the present invention, the "C₁₋₃ alkyl group" is a straight or branched chain alkyl group having 1 to 3 carbon atoms and includes, for example, a methyl, ethyl, n-propyl or isopropyl group. In R^{4c}, a methyl group or ethyl group is preferred.

In the present invention, the "C₁₋₄ alkyl group" is a straight or branched chain alkyl group having 1 to 4 carbon atoms and includes, for example, the C₁₋₃ alkyl group mentioned above or n-butyl, isobutyl, s-butyl or tert-butyl group. In R^{2a}, R^{3a}, R^{4a}, R^{2b}, R^{3b} and R^{4b}, a methyl group, an ethyl group or an isobutyl group is preferred.

In the present invention, the "C₁₋₆ alkyl group" is a straight or branched chain alkyl group having 1 to 6 carbon atoms, and includes, for example, the C₁₋₄ alkyl group mentioned above or a n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl or 2-ethylbutyl group. In R^{1a}, R^{5a}, R^{1b}, R^{5b}, and R^{6b}, straight or branched chain alkyl groups having 1 to 4 carbon atoms are preferred.

The "C₁₋₆ alkoxy group" is a group having an oxygen atom attached to a C₁₋₆ alkyl group mentioned above and includes, for example, methoxy, ethoxy, n-propoxy, isopropoxy, n-butoxy, isobutoxy, s-butoxy, tert-butoxy, n-pentoxy, and n-hexyloxy groups. In R^{5b} and R^{6b}, straight or branched chain alkoxy groups having 1 to 4 carbon atoms are preferred, and a methoxy group is more preferred. In R^{1c}, straight or branched chain alkoxy groups having 1 to 4 carbon atoms are preferred, and an ethoxy or methoxy group is more preferred.

The "C₁₋₄ alkylene group" is a straight or branched chain alkylene group having 1 to 4 carbon atoms and includes, for example, methylene, methylmethylene, ethylene, trimethylene, tetramethylene, 1-methyltrimethylene, 2-methyltrimethylene or 3-methyltrimethylene groups. In X^{2b}, a methylene group is preferred.

The "C₁₋₄ oxyalkylene group" is a group having an oxygen atom at one end of the C₁₋₄ alkylene group mentioned above and includes, for example, an oxymethylene, oxymethylmethylene, oxyethylene, oxytrimethylene or oxytetramethylene group. In X^{2b}, an oxyethylene group or an oxymethylene group is preferred.

The "C₁₋₄ thioalkylene group" is a group having a sulfur atom at one end of the C₁₋₄ alkylene group mentioned above and includes, for example, a thiomethylene, thiomethylmethylene or thioethylene group. In X^{2b}, a thioethylene or thiomethylene group is preferred.

The "halogen atom" is a fluorine atom, a chlorine atom, a bromine atom or an iodine atom. In R^{1a}, R^{1b}, R^{5b}, R^{6b}, R^{2c} and R^{3c}, a bromine atom, a chlorine atom, or a fluorine atom is preferred, and a bromine atom or a chlorine atom is more preferred.

The "C₁₋₄ alkylthio group" is a group having a sulfur atom attached to the C₁₋₄ alkyl group mentioned above and includes, for example, a methylthio, ethylthio, n-propylthio, isopropylthio, n-butylthio, isobutylthio, s-butylthio or tert-butylthio group. In R^{5a}, a n-propylthio group is preferred.

In the present invention, the "C₁₋₃ haloalkyl group" is the above-mentioned C₁₋₃ alkyl group which is substituted with a halogen atom(s), and includes, for example, a chloromethyl, trifluoromethyl, trichloromethyl, difluoromethyl, dichloromethyl, dibromomethyl, fluoromethyl, 2,2,2-trifluoroethyl, 2,2,2-trichloroethyl, 2-bromoethyl, 2-chloroethyl, 2-fluoroethyl, 2-iodoethyl or 3-chloropropyl group. In R^{4c}, chloromethyl or chloroethyl group is preferred.

In the present invention, the "C₁₋₃ aminoalkyl group" is the above-mentioned C₁₋₃ alkyl group which is substituted with an amino group, and includes, for example, an aminomethyl, aminoethyl or aminopropyl group. In R^{4c}, an aminoethyl group is preferred.

In the present invention, X^{a} and X^{1b} are the same or different, and each is preferably a sulfur atom.

In the present invention, X^{2b} is preferably a C₁₋₄ oxyalkylene group.

In the present invention, R^{1a} and R^{1b} are the same or different, and each represents preferably an amino group (said amino group may optionally be substituted with C₁₋₆ alkyl group(s)) and more preferably an amino group.

In the present invention, R^{2a} is preferably a hydrogen atom.

In the present invention, R^{3a}, R^{2b} and R^{3b} are the same or different, and each represents preferably a C₁₋₄ alkyl group.

In the present invention, R^{4a} and R^{4b} are the same or different, and each represents preferably a methyl group or an ethyl group.

In the present invention, R^{5a} is preferably a C₁₋₆ alkyl group or a C₁₋₆ alkylthio group, and more preferably a C₁₋₄ alkyl group or a C₁₋₄ alkylthio group.

In the present invention, R^{5b} and R^{6b} are the same or different, and each represents preferably a C₁₋₆ alkyl group or an amino group (said amino group may optionally be substituted with C₁₋₆ alkyl group(s)); more preferably a C₁₋₄ alkyl group or an amino group (said amino group may optionally be substituted with C₁₋₄ alkyl group(s)).

In the present invention, R^{1c} is preferably a methoxy group or an ethoxy group.

In the present invention, R^{2c} and R^{3c} are the same or different, and each represents preferably a thiazolyl group (said thiazolyl group may optionally be substituted with one methyl group or one methoxy group), an ethynyl group or a halogen atom, more preferably a thiazolyl group (said thiazolyl group may optionally be substituted with one methyl group), an ethynyl group, a chlorine atom or a fluorine atom.

In the present invention, R^{4c} is preferably a C₁₋₃ aminoalkyl group or a hydrogen atom, more preferably an aminoethyl group or a hydrogen atom.

In the present invention, n^{c} is preferably 1 or 2.

In the present invention, "Diabetes Mellitus" indicates the disease described in Journal of Japan Diabetes Society, 42 (5), 385-404 (1999), Diabetes Care, 20, 1183-1197, and Diabetes Medicine, 15, 539-553, and involves, for example, type I and type II diabetes mellitus and so forth.

In the present invention, "Prevention of the Crisis of Diabetes Mellitus" indicates prevention of the crisis of diabetes mellitus or the delayed onset of diabetes mellitus. It also involves prevention of the crisis of the disease or delayed onset of the disease by improvement of preceding states of diabetes mellitus (for example, impaired glucose tolerance). As indicators of diabetes mellitus, there are increase in the blood glucose level and prevention of increase of glycosylated hemoglobin or delayed onset of the increase in glycosylated hemoglobin, an indicator of the regulation of blood glucose level for the middle or a long term.

In the present invention, "IGT" indicates impaired glucose tolerance, which is, for example, the disease with postcibal higher blood glucose level than that in healthy subjects, and IGT is known to advance, in the future, to diabetes mellitus, arteriosclerosis and so forth at a high risk.

In the present invention, "diseases related to impaired glucose tolerance" indicates the diseases which are known to be advanced from impaired glucose tolerance, and include, for example, diabetes mellitus, diabetic complications, arteriosclerosis, macroangiopathy, hypertension and so forth.

When the FBPase inhibitor of this invention has a basic group, it can be converted to an acid addition salt according to conventional techniques. Such salts includes salts of a hydrohalogenic acid such as hydrofluoric acid, hydrochloric acid, hydrobromic acid and hydroiodic acid; salts of an inorganic acid such as a nitrate, perchlorate, sulfate and phosphate; salts of a lower alkane sulfonic acid such as methanesulfonic acid, trifluoromethanesulfonic acid, and ethanesulfonic acid; salts of an arylsulfonic acid such as benzenesulfonic acid and p-toluenesulfonic acid; salts of an amino acid such as glutamic acid and aspartic acid; and salts of a carboxylic acid such as acetic acid, fumaric acid, tartaric acid, oxalic acid, maleic acid, malic acid, succinic acid, benzoic acid, mandelic acid, ascorbic acid, lactic acid, gluconic acid and citric acid; and preferably salts of a hydrohalogenic acid.

The FBPase inhibitors, which are the active ingredients for pharmaceutical agents in this invention, can exist in the form of stereoisomeres and geometrical isomers. The present invention encompasses individual stereoisomers and geometrical isomers and mixtures thereof.

The FBPase inhibitors, which are the active ingredients for pharmaceutical agents in this invention, can exist in the form of hydrates and solvates. The present invention encompasses individual hydrates and solvates and mixtures thereof.

Compounds (for example amide derivatives, so-called pro-drugs) that can be converted by metabolism in the living body into the FBPase inhibitors, which are the active ingredients for pharmaceutical agents of this invention, are encompassed in the present invention.

In the present invention, one or more FBPase inhibitors can be used as the pharmaceutical agent.

The FBPase inhibitors, pharmacologically acceptable salts thereof and esters thereof of this invention can be administered in various dosage forms. There is no particular limitation on the dosage forms and the dosage form is determined by the formulations, the age, sexuality and other conditions of the patient, the degree of the symptoms and the like. For example, dosage forms for oral administration include tablets, pills, powders, granules, syrups, solutions, suspensions, emulsions, and capsules. In the case of injections an active ingredient alone can be intravenously administered or a mixture of an active ingredient with conventional adjuvant solutions such as glucose or amino acid can be intravenously administered and, if necessary, an active ingredient alone can also intramuscularly, intracutaneously, subcutaneously or intraabdominaly administered. Suppositories are administered into the rectum. Preferably they are orally administered.

These dosage forms can be prepared according to conventional techniques using adjuvants commonly used in the art such as excipients, binding agents, disintegrating agents, lubricating agents, solubilizing agents, corrigents, flavoring and perfuming agents and coating agents.

The tablets can be prepared using carriers commonly used in the art, for example, excipients such as lactose, sucrose, sodium chloride, glucose, urea, starch, calcium carbonate, kaolin, crystalline cellulose, silicic acid and the like; binding agents such as water, ethanol, propanol, simple syrup, glucose solution, starch solution, gelatin solution, carboxymethylcellulose; shellac, methylcellulose, potassium phosphate, polyvinylpyrrolidone and the like; disintegrating agents such as dry starch, sodium alginate, agar powder, laminaran powder, sodium hydrogencarbonate, calcium carbonate, polyoxyethylene sorbitan fatty acid esters, sodium lauryl sulfate, stearic acid mono-glyceride, starch, lactose and the like; disintegration suppressants such as sucrose, stearin, cacao butter, hydrogenated oil and the like; absorption facilitators such as quaternary ammonium bases, sodium lauryl sulfate and the like; wetting agents such as glycerin, starch and the like, absorbents such as starch, lactose, kaolin, bentonite, colloidal silcic acid and the like; and lubricating agents such as purified talc, stearates, boric acid powder, polyethyleneglycol and the like. The tablets, if necessary, can be converted to coated tablets, for example, sugar-coated tablets, gelatin-coated tablets, enteric-coated tablets, film-coated tablets, double-coated tablets or multilayer-coated tablets.

The pills can be prepared using carriers commonly used in the art, for example, excipients such as glucose, lactose, starch, cacao butter, hydrogenated vegetable oil, kaolin, talc and the like; binding agents such as acasia powder, tragacanth powder, gelatin, ethanol and the like; and disintegrating agents such as laminaran agar and the like.

The suppositories can be prepared using carriers commonly used in the art, for example polyethyleneglycol, cacao butter, higher alcohols, esters of higher alcohol, gelatin, semi-synthetic glycerides and the like.

When the injections are prepared, solubilizing and suspending agents are preferably sterilized and are isotonic to the blood. The solutions, emulsions and suspensions for the injections can be prepared using diluents commonly used in the art, for example, water, ethyl alcohol, propylene glycol, ethoxylated isostearyl alcohol, polyoxylated isostearyl alcohol, polyoxyethylene sorbitan fatty acid esters and the like. In addition, in this case an adequate amount of sodium chloride, glucose or glycerin may be contained in the injection solutions in order to prepare isotonic solutions. Solubilizing agents, buffering agents and soothing agents can also be added to the solutions for the injections.

Furthermore, if necessary, coloring agents, preserving agents, flavoring agents, tasting agents, sweetening agents and the like and other medicaments may be optionally contained.

The amount of the active ingredient in the above dosage form is not particularly limited and is appropriately selected in a wide range. It is usually in the range from 1 to 70% (w/w) of the total weight of composition of dosage form, preferably from 1 to 30% (w/w).

The doses will vary depending on a variety of factors such as the symptoms, age and body weight of the patient and the route of administration and dosage form. A suitable dosage level is from 0.001 mg (preferably 0.01 mg and more preferably 0.1 mg) per day as a lower limit to 2000 mg (preferably 200 mg and more preferably 20 mg) per day as an upper limit for an adult human. The suitable dosage can be administered from one to several times throughout the day.

### [Brief explanation of the drawings]

Figure 1 represents the changes of blood glucose level following administration of Compound A (the FBPase inhibitor).
Figure 2 represents the changes of glycosylated hemoglobin ratio following administration of Compound A (the FBPase inhibitor).
Figure 3 represents the changes of plasma triglyceride level following administration of Compound A (the FBPase inhibitor).
Figure 4 represents the changes of plasma lactate level following administration of Compound A (the FBPase inhibitor).
Figure 5 represents the changes of area under the curves (AUCs) for blood glucose levels following administration of Compound A (the FBPase inhibitor).
Figure 6 represents the changes of area under the curves (AUCs) for blood glucose levels following administration of Compound B (the FBPase inhibitor).

### [Best mode for carrying out the invention]

### [Examples]

### (Example 1)

### Preventative effects on diabetes mellitus of administering 2-amino-5-isobutyl-4-{2-[5-(N,N'-bis((S)-1-ethoxycarbonyl)ethyl)phosphonamido]furanyl}thiazole (Compound A).

6-week-old GK rats (Charles River Japan, Inc.) which were prior to the crisis of diabetes mellitus, were used. Compound A was mixed with food powder for the rat (F2, Funabashi Farm Co.,) at concentrations of 0.04%, 0.2% and 1.0% (w/w) and was orally administered to 4-6 rats per group for 8 weeks ad libitum (the mean amount of food intake per rat: 17.3 g/day).

The rats in the control group were fed with only food powder.

Blood from each rat was collected from the tail vein every 2 weeks, and the blood glucose level, plasma lactate level and plasma triglyceride level were determined. Blood glucose level was determined by electric potential measurement method based on glucose oxidase immobilized electrode using Glucoloader GXT (A&T Corp.). Plasma lactate level was determined using a spectrophotometric method with Lactate Reagent (Sigma-Aldrich Japan K. K.), and plasma triglyceride level was determined using a spectrophotometric method with Triglyceride E Test Wako (Wako Pure Chemical Industries, Ltd.). When the blood was collected after the drug administration period and the drug withdrawal period for 2 weeks, glycosylated hemoglobin ratio was also determined with DCA-2000 (Bayer Medical Co., Ltd.).

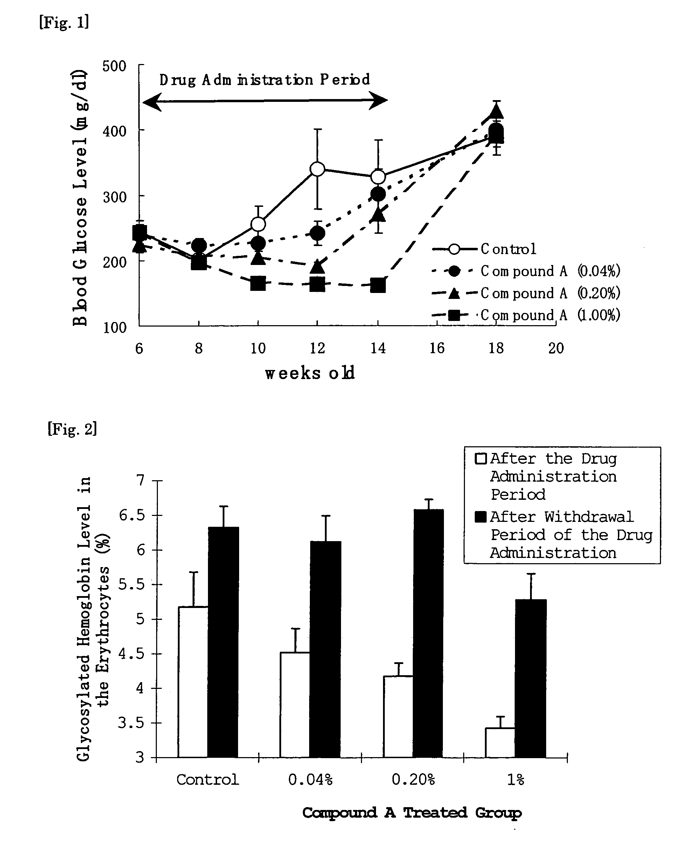

The results are demonstrated in the following Fig. 1-4.

As shown in Fig. 1, it is clear in the control group that the blood glucose level started to increase at 10-weeks-old (4 weeks after initiation of the experiment), and attained a level higher than 300 mg/dl, which indicated the crisis of diabetes mellitus, at 12- to 14-week-old (6-8 weeks after initiation of the experiment). On the other hand, in the group treated with Compound A, the increase in the blood glucose level was inhibited in a dose-dependent manner. Further, after the drug administration period was terminated, the blood glucose level increased and attained the same maximum level as that in the control group, which was identified as crisis of diabetes mellitus. Thus, the crisis of diabetes mellitus in the GK rat was clearly inhibited by Compound A.

As demonstrated in Fig. 2, the inhibitory effects of Compound A on the crisis of diabetes mellitus was also observed when the glycosylated hemoglobin ratio, which was an indicator of the blood glucose regulation, was used as an indicator. That is, the glycosylated hemoglobin ratio in rats treated with Compound A was decreased in a dose-dependent manner, suggesting that the crisis of diabetes mellitus was suppressed. Further, when Compound A was withdrawn from administration, the glycosylated hemoglobin ratio was increased to the same level as that in the control group. Thus, it was clearly shown that the blood glucose level was significantly suppressed following administration of Compound A.

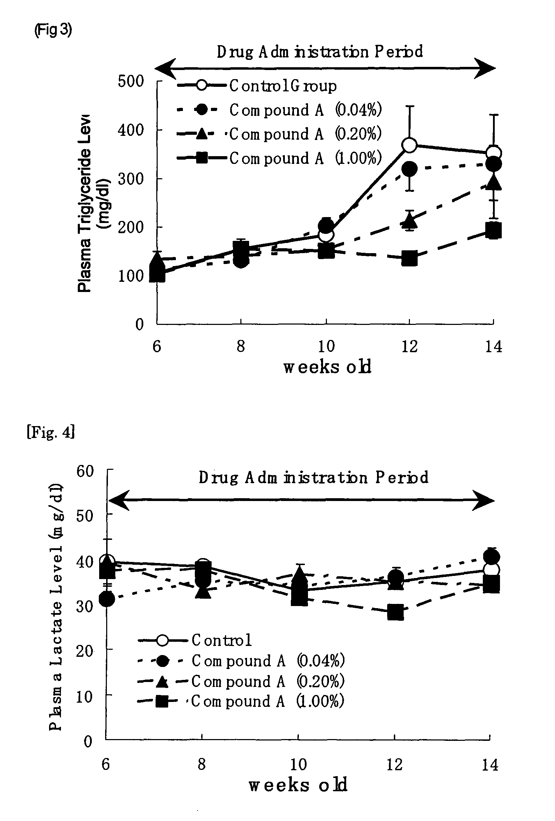

In this experiment, as shown in Fig. 3, increase in plasma triglyceride level, which was an indicator of hyperlipidemia, was observed in the control group of the GK rats, as well as the crisis of diabetes mellitus. In the group treated with Compound A, however, the plasma triglyceride level was not increased as much as those in the control rats or was prevented from increase during the administration period. It is well known that hyperlipidemia is a risk factor of diabetes mellitus, as well as a risk factor of cardiovascular disorders, such as heart disease and cerebral stroke infarction. Thus the results presented in Fig. 3 showed clearly that FBPase inhibitors suppressed not only crisis of diabetes mellitus but were also effective in the reduction of risk factors of metabolic diseases and macroangiopathy associated with hyperlipidemia.

It is generally believed that hepatic glucose production is enhanced more significantly in patients with diabetes mellitus than that in the healthy subjects. The hepatic glucose production is related to the hepatic gluconeogenesis and glycogenolysis.

In the diabetic patient, the hepatic gluconeogenesis is the largest contributor to the enhanced hepatic glucose production. Several rate-limiting enzymes regulate the hepatic gluconeogenesis. The FBPase, one of the rate-limiting enzymes, is localized in the liver and the kidney, and is an essential enzyme for the gluconeogenesis.

That is, the diabetes mellitus is closely related to the FBPase activity in the liver. Since most orally administered FBPase inhibitors were exposed to the liver via the portal vein, the FBPase inhibitor suppressed potently the hepatic gluconeogenesis, and resulted in prevention of crisis of the diabetes mellitus. Further, in the present experiments, it was clearly demonstrated that the FBPase inhibitor was effective in metabolic disorders and macroangiopathy associated with hyperlipidemia.

As shown in Fig. 4, the plasma lactate level was roughly stable during the administration period of Compound A, and the group treated with Compound A was not significantly different from the control group. Thus, it was suggested that Compound A could be used without any adverse events to increase in the lactate level, unlike metformin. Further, no animal suffered gastrointestinal symptoms such as diarrhea in all the groups examined. Thus, Compound A is considered not to elicit any adverse events in the gastrointestine, unlike metformin.

As one of the mechanisms of the blood glucose lowering effects of metformin, suppression of hepatic gluconeogenesis has been reported (Diabetes Research and Clinical Practice, 19, 11-17, 1993: ibid, 19, 49-58, 1993). Further, the inhibitory mechanisms of hepatic gluconeogenesis by metformin are considered to be due to uptake inhibition of alanine, one of the substrates for gluconeogenesis, into the hepatocytes, and also due to reducing the conversion of pyruvate, another substrate for hepatic gluconeogenesis, to lactate. Hence metformin is considered to induce adverse events, such as lactic acidosis. In addition, since metformin tends to be accumulated in the digestive organs, metformin could cause gastrointestinal symptoms easily. Thus FBPase inhibitors have different mechanisms of action from those of metformin. FBPase inhibitors, of which Compound A is a representative, are considered not to cause any adverse events, such as lactic acidosis and gastrointestinal symptoms elicited by metformin.

### (Example 2)

### Improving effects on impaired glucose tolerance, in the precritical state of diabetes mellitus, of 2-amino-5-isobutyl-4-{2-[5-(N,N'-bis((S)-1-ethoxycarbonyl)ethyl)phosphonamido]furanyl}thiazole (Compound A)

6-week-old GK rats (Charles River Japan, Inc.) or ZDF rats (Charles River Japan, Inc.), which show IGT symptoms, in the precritical state of diabetes mellitus, are fasted overnight. Compound A is orally administered and 2 g/kg of 50% glucose solution is orally administered 1 hour after the administration of Compound A. The blood is collected from the tail vein 1 hour before, just before administration of glucose, 30, 60, 120 and 240 minutes after administration of glucose solution. Blood glucose level is determined by electric potential measurement method based on glucose oxidase immobilized electrode using Glucoloader GXT (A&T Corp.).

### (Example 3)

### Improving effects on impaired glucose tolerance of 2-amino-5-isobutyl-4-{2-[5-(N,N'-bis((S)-1-ethoxycarbonyl)ethyl)-phosphonamido]furanyl}thiazole (Compound A)

6-week-old GK rats of (Charles River Japan, Inc.) which show IGT symptoms, but not diabetes mellitus, were fasted overnight. Compound A was orally administered at dosages of 10, 20 or 40 mg/kg. Two gram per kilogram of 50% (w/w) glucose solution was orally administered 1 hour after the administration of Compound A. The rats in the control group were administered orally 2 g/kg of 50% (w/w) glucose solution only.

The blood was collected from the tail vein just before and 0.5, 1, 2 and 4 hours after the glucose administration. Blood glucose level was determined by electric potential measurement method based on a glucose oxidase immobilized electrode using Glucoloader GXT (A&T Corp.).

On the abscissas, the time after the administration of Compound A was plotted, and on the ordinate, the blood glucose level of each individual was plotted. Each point was drawn with a straight line. The areas under the curves (AUCs) for the blood glucose levels of the graph obtained were calculated.

Using the areas under the curves (AUCs) for the blood glucose levels, the average value and standard error of each group were calculated.

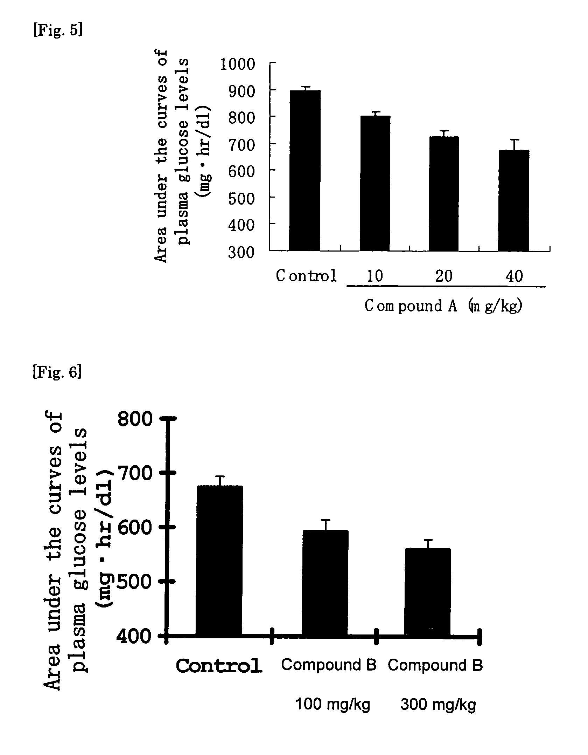

The result is demonstrated in the following Fig. 5.

As shown in Fig. 5, the AUCs of the blood glucose levels were decreased by the treatment of Compound A in a dose-dependent manner. Since the AUCs of the blood glucose levels are an indicator of the blood glucose regulation after the glucose administration, these results indicate that Compound A improved impaired glucose tolerance, suggesting that FBPase inhibitors, of which Compound A is a representative, are useful as therapeutic and/or preventive agents for IGT.

### (Example 4)

### Improving effects of (6,7-diethoxy-quinazolin-4-yl)-[3-(2-methyl-thiazol-4-yl)phenyl]amine (Compound B) on impaired glucose tolerance

6-week-old GK rats (Charles River Japan, Inc.) which show IGT symptoms, but not diabetes mellitus, were fasted overnight. Compound B was orally administered at dosages of 100 mg/kg or 300 mg/kg. Two gram per kilogram of 50% (w/w) glucose solution was orally administered 1 hour after the administration of Compound B. The rats in the control group were administered orally 2 g/kg of 50% glucose solution.

The blood was collected from the tail vein just before and 0.5, 1 and 2 hours after the glucose administration. Blood glucose level was determined by electric potential measurement method based on a glucose oxidase immobilized electrode using Glucoloader GXT (A&T Corp.).

On the abscissas, the time after the administration of Compound B was plotted, and on the ordinate, the blood glucose level of each individual was plotted. Each point was drawn with a straight line. The areas under the curves (AUCs) for the blood glucose levels of the graph obtained were calculated.

Using the areas under the curves (AUCs) for the blood glucose levels, the average value and standard error of each group were calculated.

The result is demonstrated in the following Fig. 6.

As shown in Fig. 6, the AUCs of the blood glucose levels were decreased by the treatment of Compound B in a dose-dependent manner. Since the AUCs of the blood glucose levels are an indicator of the blood glucose regulation after the glucose administration, these results indicate that Compound B improved impaired glucose tolerance, suggesting that FBPase inhibitors, of which Compound B is a representative, are useful as therapeutic and/or preventive agents for IGT.

Generally, there are 3 main ways to improve the impaired glucose tolerance in diabetic patients, that are, firstly enhancement of glucose utilization in the body, secondly facilitation of glucose excretion, and finally inhibition of hepatic glucose production. Further, hepatic glucose production is related to hepatic gluconeogenesis, and FBPase inhibitors are believed to inhibit hepatic gluconeogenesis by inhibition of FBPase, a rate-limiting enzyme in hepatic gluconeogenesis. Thus, the hepatic glucose production is considered to be suppressed by FBPase inhibitors which inhibit FBPase activity, resulting in amelioration of IGT.

Actually, from the results of Example 3 and Example 4, FBPase inhibitors having different structures showed improved activity for IGT similarly. That is, such improved activity for IGT is considered not to be a particular activity of the specific compounds but an activity derived from the inhibition activity of FBPase as described above. Accordingly, compounds having inhibitory effect on FBPase are useful as therapeutic and/or preventive agents for IGT.

### (Formulation examples)

| (1) capsule | |
|---|---|
| compound A | 10 mg |
| lactose | 110 mg |
| corn starch | 58 mg |
| magnesium stearate | 2 mg |
| total | 180 mg |

The components shown above are adequately mixed and the mixture is passed through a 60 mesh (Tyler standard) sieve. The resulting powder (180 mg) is injected into a gelatin capsule (No. 3) to give the desired capsule.

| (2) tablet | |
|---|---|
| compound A | 10 mg |
| lactose | 85 mg |
| corn starch | 34 mg |
| microcrystalline cellulose | 20 mg |
| magnesium stearate | 1 mg |
| total | 150 mg |

The components shown above are adequately mixed and the mixture is pressed by a tablet machine to give the desired tablet (150 mg). The tablet, if necessary, can be coated with sugar or film.

| (3) granules | |
|---|---|
| compound A | 10 mg |
| lactose | 839 mg |
| corn starch | 150 mg |
| hydroxypropylcellulose | 1 mg |
| total | 1000 mg |

The components shown above are adequately mixed and the mixture is wetted with pure water. The resulting product is granulated by a basket-type granulating machine and dried to give the desired granules.

### [Industrial applicability]

The pharmaceutical agents containing FBPase inhibitors of the present invention exert excellent preventative effects on diabetes mellitus. Further, they lack adverse events that were elicited by metformin (increase in plasma lactate level and gastrointestinal symptoms). Thus they are useful as preventative agents of diabetes mellitus with excellent safety and as a result, they can be used for a long period.

Moreover, the pharmaceutical agents containing FBPase inhibitors of the present invention are also useful as therapeutic and/or preventative agents for IGT, which is widely observed as a preceding process to diabetes mellitus.

Then, the pharmaceutical agents containing FBPase inhibitors of the present invention are also useful as preventative agents for diseases related to IGT such as diabetes mellitus, diabetic complications, hypertension, arteriosclerosis, macroangiopathy and the like.

In addition, since FBPase inhibitors blocked increased plasma triglyceride level, one of indicators of hyperlipidemia, they are useful as preventative agents for diseases in which hyperlipidemia (main symptoms; for example, high plasma level of acylglycerol, hypercholesterolemia and so forth) is a risk factor (for example, macroangiopathy).

## Claims

1. A preventative agent for diabetes mellitus, which contains one or more FBPase inhibitors.

2. A therapeutic agent for impaired glucose tolerance, which contains one or more FBPase inhibitors.

3. A preventative agent for impaired glucose tolerance, which contains one or more FBPase inhibitors.

4. A therapeutic agent for impaired glucose tolerance in the precritical state of diabetes mellitus which contains one or more FBPase inhibitors.

5. A preventative agent for impaired glucose tolerance in the precritical state of diabetes mellitus which contains one or more FBPase inhibitors.

6. A preventative agent for hyperlipidemia or arteriosclerosis, which contains one or more FBPase inhibitors.

7. A preventative agent for diseases related to impaired glucose tolerance, which contains one or more FBPase inhibitors.

8. A preventative agent according to claim 7 wherein the diseases related to impaired glucose tolerance are diabetes mellitus, diabetic complications, hypertension, arteriosclerosis or macroangiopathy.

9. A preventative agent according to claim 7 wherein the diseases related to impaired glucose tolerance are macroangiopathy or arteriosclerosis.

10. A therapeutic agent for impaired glucose tolerance which contains one or more FBPase inhibitors, and said agent has no adverse gastrointestinal events.

11. A preventative agent for diseases related to impaired glucose tolerance which contains one or more FBPase inhibitors, and said agent has no adverse gastrointestinal events.

12. A preventative agent according to claim 11 wherein the diseases related to impaired glucose tolerance are macroangiopathy or arteriosclerosis.

13. A therapeutic or preventative agent according to claims 1 to 12 wherein the FBPase inhibitor is a compound of the following formula (I) or a pharmacologically acceptable salt thereof: 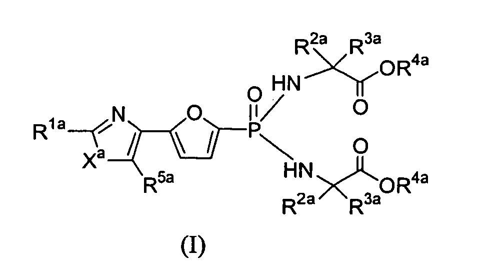 wherein X^{a} represents a nitrogen atom, an oxygen atom or a sulfur atom; R^{1a} represents a hydrogen atom, a halogen atom, a C₁₋₆ alkyl group, or an amino group (said amino group may optionally be substituted with one or two C₁₋₆ alkyl groups); R^{2a} and R^{3a} are the same or different, and each represents a hydrogen atom or a C₁₋₄ alkyl group; R^{4a} represents a C₁₋₄ alkyl group; and R^{5a} represents a hydrogen atom, a C₁₋₆ alkyl group or a C₁₋₆ alkylthio group.

14. A therapeutic or preventative agent according to claims 1 to 12 wherein the FBPase inhibitor is a compound of the following formula (Ia) or a pharmacologically acceptable salt thereof: 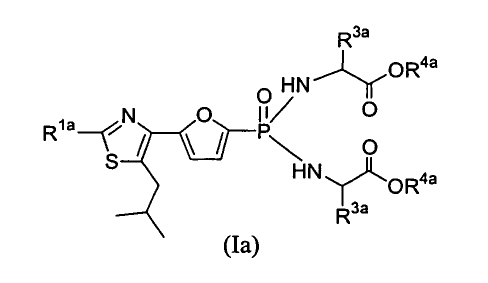 wherein R^{1a} represents an amino group (said amino group may optionally be substituted with one or two C₁₋₆ alkyl groups); R^{3a} represents a hydrogen atom or a C₁₋₆ alkyl group; and R^{4a} represents a C₁₋₄ alkyl group.

15. A therapeutic or preventative agent according to claims 1 to 12 wherein the FBPase inhibitor is a compound of the following formula (II) or a pharmacologically acceptable salt thereof: 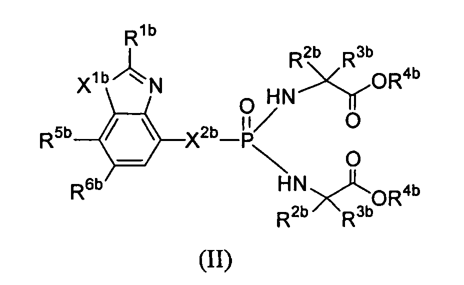 wherein X^{1b} represents an oxygen atom or a sulfur atom; X^{2b} represents a C₁₋₄ alkylene group, a C₁₋₄ oxyalkylene group (with the proviso that the phosphorous atom is attached to a carbon atom), or a C₁₋₄ thioalkylene group (with the proviso that the phosphorous atom is attached to a carbon atom); R^{1b} represents a hydrogen atom, a halogen atom, a C₁₋₆ alkyl group or an amino group (said amino group may optionally be substituted with one or two C₁₋₆ alkyl groups); R^{2b} and R^{3b} are the same or different, and each represents a hydrogen atom or a C₁₋₄ alkyl group; R^{4b} represents a C₁₋₄ alkyl group; and R^{5b} and R^{6b} are the same or different, and each represents a hydrogen atom, a halogen atom, a C₁₋₆ alkyl group, a C₁₋₆ alkoxy group, or an amino group (said amino group may optionally be substituted with one or two C₁₋₆ alkyl groups).

16. A therapeutic or preventative agent according to claims 1 to 12 wherein the FBPase inhibitor is 2-amino-5-isobutyl-4-{2-[5-(N,N'-bis((S)-1-ethoxycarbonyl)ethyl)-phosphonamido]furanyl}thiazole, 2-amino-5-isobutyl-4-{2-[5-(O-(2-bis(N-(1-methyl-1-ethoxycarbonyl)ethyl)phosphonamido)furanyl)thiazole, 2-amino-5-propylthio-4-{2-[5-(N,N'-(1-(S)ethoxycarbonyl)ethyl)phosphonamido]-furanyl}thiazole, 2-amino-5-propylthio-4-{2-[5-(N,N'-(1-methyl-ethoxycarbonyl)ethyl)phosphonamido]furanyl}thiazole, or a pharmacologically acceptable salt thereof.

17. A therapeutic or preventative agent according to claims 1 to 12 wherein the FBPase inhibitor is a compound of the following formula (III') or a pharmacologically acceptable salt thereof: 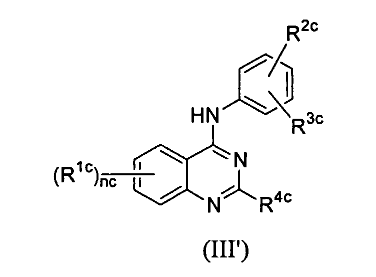 wherein R^{1c} represents a C₁₋₄ alkoxy group; R^{2c} and R^{3c} are the same or different, and each represents a thiazolyl group (said thiazolyl group may optionally be substituted with one methyl group or one methoxy group), an ethynyl group, a hydrogen atom, or a halogen atom; R^{4c} represents a C₁₋₃ alkyl group (said alkyl group may optionally be substituted with one imidazolyl group), a C₁₋₃ haloalkyl group, a C₁₋₃ aminoalkyl group or a hydrogen atom; and n^{c} represents an integer of from 1 to 3.

18. A therapeutic or preventative agent according to claims 1 to 12 wherein the FBPase inhibitor is a compound of the following formula (IIIa') or a pharmacologically acceptable salt thereof: 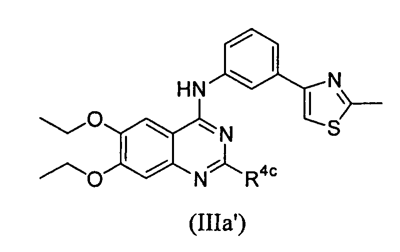 wherein R^{4c} represents a C₁₋₃ alkyl group (said alkyl group may optionally be substituted with one imidazolyl group), a C₁₋₃ haloalkyl group, a C₁₋₃ aminoalkyl group or a hydrogen atom.

19. A therapeutic or preventative agent according to claims 1 to 12 wherein the FBPase inhibitor is a compound of the following formula (III) or a pharmacologically acceptable salt thereof: 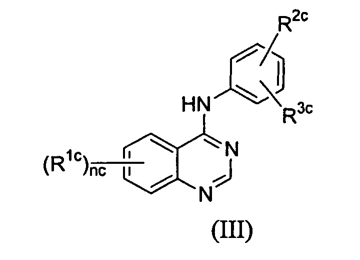 wherein R^{1c} represents a C₁₋₄ alkoxy group; R^{2c} and R^{3c} are the same or different, and each represents an ethynyl group, a hydrogen atom or a halogen atom; and n^{c} represents an integer of from 1 to 3.

20. A therapeutic or preventative agent according to claims 1 to 12 wherein the FBPase inhibitor is (6,7-dimethoxy-quinazolin-4-yl)-(3-ethynyl-4-fluorophenyl)amine of the following formula (IIIa), 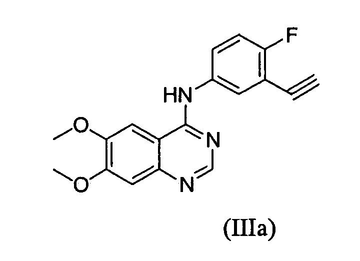 (6,7-diethoxy-quinazolin-4-yl)-[3-(2-methyl-thiazol-4-yl)phenyl]amine of the following formula (IIIb), 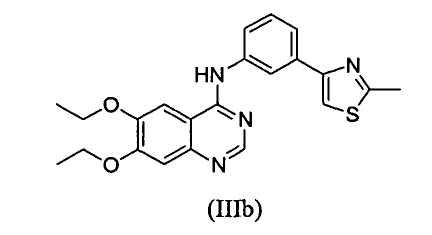 or (2-aminoethyl-6,7-diethoxy-quinazolin-4-yl)-[3-(2-methyl-thiazol-4-yl)phenyl]amine of the following formula (IIIc), or a pharmacologically acceptable salt thereof.

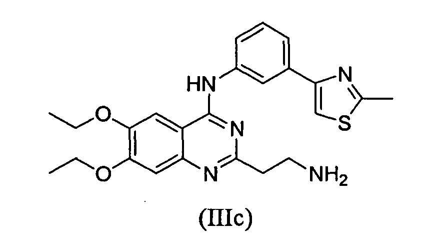

21. A therapeutic or preventative agent according to claims 1 to 12 wherein the FBPase inhibitor is 2-(4-cyanophenyl)-2-[(3S,11aS)-3-cyclohexylmethyl-8-hydroxy-1,4-dioxo-1,2,3,4,6,11,11a-octahydropyrazino[1,2-b]isoquinolin-2-yl]-N-[2-(4-hydroxyphenyl)ethyl]acetamide of the following formula (IV) or a pharmacologically acceptable salt thereof.

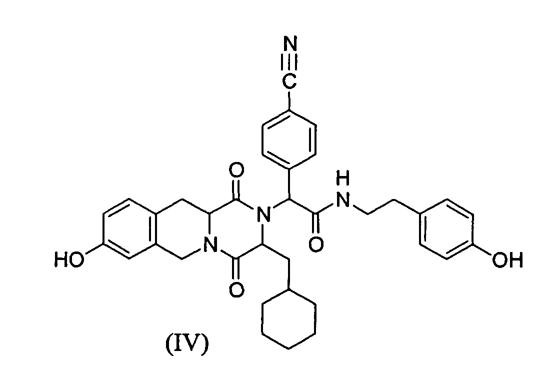

22. A pharmaceutical composition for the prevention of diabetes mellitus, which contains an FBPase inhibitor as an active ingredient.

23. A pharmaceutical composition for the treatment of impaired glucose tolerance, which contains one or more FBPase inhibitors as active ingredients.

24. A pharmaceutical composition for the prevention of diseases related to impaired glucose tolerance, which contains one or more FBPase inhibitors as active ingredients.

25. A pharmaceutical composition according to claim 24 wherein the diseases related to impaired glucose tolerance are macroangiopathy or arteriosclerosis.

26. A pharmaceutical composition for the prevention or treatment of diseases according to claims 22 to 25 wherein the FBPase inhibitor is a compound of the following formula (I) or a pharmacologically acceptable salt thereof: 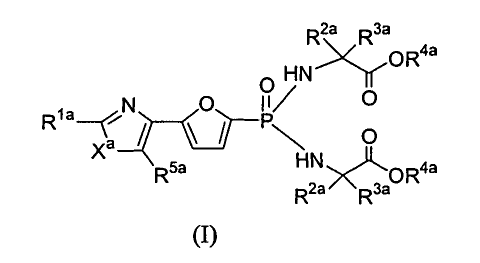 wherein X^{a} represents a nitrogen atom, an oxygen atom or a sulfur atom; R^{1a} represents a hydrogen atom, a halogen atom, a C₁₋₆ alkyl group, or an amino group (said amino group may optionally be substituted with one or two C₁₋₆ alkyl groups); R^{2a} and R^{3a} are the same or different, and each represents a hydrogen atom or a C₁₋₄ alkyl group; R^{4a} represents a C₁₋₄ alkyl group; and R^{5a} represents a hydrogen atom, a C₁₋₆ alkyl group or a C₁₋₆ alkylthio group.

27. A pharmaceutical composition for the prevention or treatment of diseases according to claims 22 to 25 wherein the FBPase inhibitor is a compound of the following formula (Ia) or a pharmacologically acceptable salt thereof: 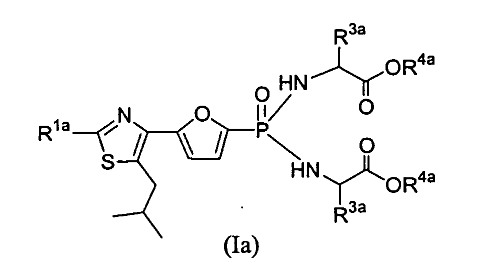 wherein R^{1a} represents an amino group (said amino group may optionally be substituted with one or two C₁₋₆ alkyl groups); R^{3a} represents a hydrogen atom or a C₁₋₄ alkyl group; and R^{4a} represents a C₁₋₄ alkyl group.

28. A pharmaceutical composition for the prevention or treatment of diseases according to claims 22 to 25 wherein the FBPase inhibitor is a compound of the following formula (II) or a pharmacologically acceptable salt thereof: 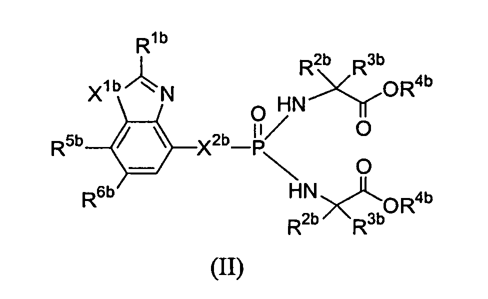 wherein X^{1b} represents an oxygen atom or a sulfur atom; X^{2b} represents a C₁₋₄ alkylene group, a C₁₋₄ oxyalkylene group (with the proviso that the phosphorous atom is attached to a carbon atom), or a C₁₋₄ thioalkylene group (with the proviso that the phosphorous atom is attached to a carbon atom); R^{1b} represents a hydrogen atom, a halogen atom, a C₁₋₆ alkyl group or an amino group (said amino group may optionally be substituted with one or two C₁₋₆ alkyl groups); R^{2b} and R^{3b} are the same or different, and each represents a hydrogen atom or a C₁₋₄ alkyl group; R^{4b} represents a C₁₋₄ alkyl group; and R^{5b} and R^{6b} are the same or different, and each represents a hydrogen atom, a halogen atom, a C₁₋₆ alkyl group, a C₁₋₆ alkoxy group, or an amino group (said amino group may optionally be substituted with one or two C₁₋₆ alkyl groups).

29. A pharmaceutical composition for the prevention or treatment of diseases according to claims 22 to 25 wherein the FBPase inhibitor is 2-amino-5-isobutyl-4-{2-[5-(N,N'-bis((S)-1-ethoxycarbonyl)ethyl)phosphonamido]furanyl}thiazole, 2-amino-5-isobutyl-4-{2-[5-(O-(2-bis(N-(1-methyl-1-ethoxycarbonyl)ethyl)-phosphonamido)furanyl)thiazole, 2-amino-5-propylthio-4-{2-[5-(N,N'-(1-(S)ethoxycarbonyl)ethyl)phosphonamido]furanyl}thiazole, 2-amino-5-propylthio-4-{2-[5-(N,N'-(1-methyl-ethoxycarbonyl)ethyl)phosphonamido]furanyl}thiazole, or a pharmacologically acceptable salt thereof.

30. Use of an FBPase inhibitor in the manufacture of a medicament for the prevention of diabetes mellitus.

31. Use of an FBPase inhibitor in the manufacture of a medicament for the treatment of impaired glucose tolerance.

32. Use of an FBPase inhibitor in the manufacture of a medicament for the prevention of diseases related to impaired glucose tolerance.

33. Use of an FBPase inhibitor in the manufacture of a medicament according to claim 32 wherein the diseases related to impaired glucose tolerance are macroangiopathy or arteriosclerosis.

34. Use of an FBPase inhibitor in the manufacture of a medicament according to claims 30 to 33 wherein the FBPase inhibitor is a compound of the following formula (I) or a pharmacologically acceptable salt thereof: 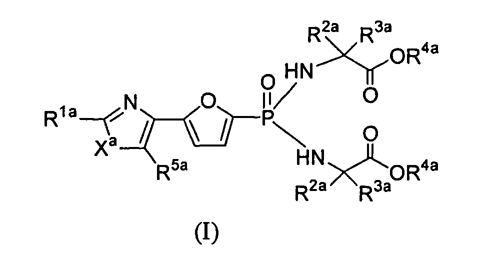 wherein X^{a} represents a nitrogen atom, an oxygen atom or a sulfur atom; R^{1a} represents a hydrogen atom, a halogen atom, a C₁₋₆ alkyl group, or an amino group (said amino group may optionally be substituted with one or two C₁₋₆ alkyl groups); R^{2a} and R^{3a} are the same or different, and each represents a hydrogen atom or a C₁₋₄ alkyl group; R^{4a} represents a C₁₋₄ alkyl group; and R^{5a} represents a hydrogen atom, a C₁₋₆ alkyl group or a C₁₋₆ alkylthio group.

35. Use of an FBPase inhibitor in the manufacture of a medicament according to claims 30 to 33 wherein the FBPase inhibitor is a compound of the following formula (Ia) or a pharmacologically acceptable salt thereof: 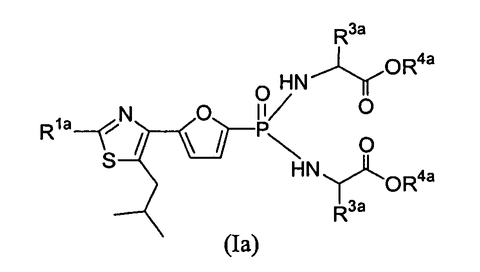 wherein R^{1a} represents an amino group (said amino group may optionally be substituted with one or two C₁₋₆ alkyl groups); R^{3a} represents a hydrogen atom or a C₁₋₄ alkyl group; and R^{4a} represents a C₁₋₄ alkyl group.

36. Use of an FBPase inhibitor in the manufacture of a medicament according to claims 30 to 33 wherein the FBPase inhibitor is a compound of the following formula (II) or a pharmacologically acceptable salt thereof: 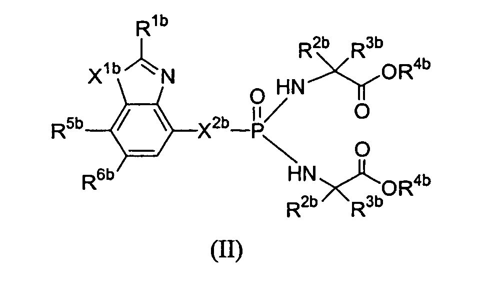 wherein X^{1b} represents an oxygen atom or a sulfur atom; X^{2b} represents a C₁₋₄ alkylene group, a C₁₋₄ oxyalkylene group (with the proviso that the phosphorous atom is attached to a carbon atom), or a C₁₋₄ thioalkylene group (with the proviso that the phosphorous atom is attached to a carbon atom); R^{1b} represents a hydrogen atom, a halogen atom, a C₁₋₆ alkyl group or an amino group (said amino group may optionally be substituted with one or two C₁₋₆ alkyl groups); R^{2b} and R^{3b} are the same or different, and each represents a hydrogen atom or a C₁₋₄ alkyl group; R^{4b} represents a C₁₋₄ alkyl group; and R^{5b} and R^{6b} are the same or different, and each represents a hydrogen atom, a halogen atom, a C₁₋₆ alkyl group, a C₁₋₆ alkoxy group, or an amino group (said amino group may optionally be substituted with one or two C₁₋₆ alkyl groups).

37. Use of an FBPase inhibitor in the manufacture of a medicament according to claims 30 to 33 wherein the FBPase inhibitor is 2-amino-5-isobutyl-4-{2-[5-(N,N'-bis((S)-1-ethoxycarbonyl)ethyl)phosphonamido]furanyl}thiazole, 2-amino-5-isobutyl-4-{2-[5-(O-(2-bis(N-(1-methyl-1-ethoxycarbonyl)ethyl)phosphonamido)furanyl)-thiazole, 2-amino-5-propylthio-4-{2-[5-(N,N'-(1-(S)ethoxycarbonyl)ethyl)-phosphonamido]furanyl}thiazole, 2-amino-5-propylthio-4-{2-[5-(N,N'-(1-methyl-ethoxycarbonyl)ethyl)phosphonamido]furanyl}thiazole, or a pharmacologically acceptable salt thereof.

38. A method for the prevention of diabetes mellitus, comprising administering a pharmacologically effective amount of an FBPase inhibitor to a warm-blooded animal.

39. A method for the treatment of impaired glucose tolerance, comprising administering a pharmacologically effective amount of an FBPase inhibitor to a warm-blooded animal.

40. A method for the prevention of diseases related to impaired glucose tolerance, comprising administering a pharmacologically effective amount of an FBPase inhibitor to a warm-blooded animal.

41. A method for the prevention of diseases related to impaired glucose tolerance by improving impaired glucose tolerance, comprising administering a pharmacologically effective amount of an FBPase inhibitor to a warm-blooded animal.

42. A method according to claim 40 or 41 wherein the diseases related to impaired glucose tolerance are macroangiopathy or arteriosclerosis.

43. A method for the prevention or treatment of diseases according to claims 38 to 42 wherein the FBPase inhibitor is a compound of the following formula (I) or a pharmacologically acceptable salt thereof: 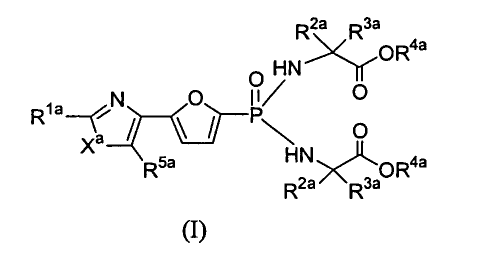 wherein X^{a} represents a nitrogen atom, an oxygen atom or a sulfur atom; R^{1a} represents a hydrogen atom, a halogen atom, a C₁₋₆ alkyl group, or an amino group (said amino group may optionally be substituted with one or two C₁₋₆ alkyl groups); R^{2a} and R^{3a} are the same or different, and each represents a hydrogen atom or a C₁₋₄ alkyl group; R^{4a} represents a C₁₋₄ alkyl group; and R^{5a} represents a hydrogen atom, a C₁₋₆ alkyl group or a C₁₋₆ alkylthio group.

44. A method for the prevention or treatment of diseases according to claims 38 to 42 wherein the FBPase inhibitor is a compound of the following formula (Ia) or a pharmacologically acceptable salt thereof: 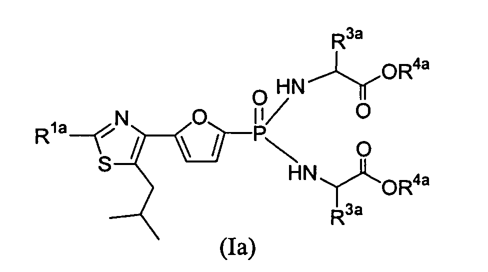 wherein R^{1a} represents an amino group (said amino group may optionally be substituted with one or two C₁₋₆ alkyl groups); R^{3a} represents a hydrogen atom or a C₁₋₄ alkyl group; and R^{4a} represents a C₁₋₄ alkyl group.

45. A method for the prevention or treatment of diseases according to claims 38 to 42 wherein the FBPase inhibitor is a compound of the following formula (II) or a pharmacologically acceptable salt thereof: 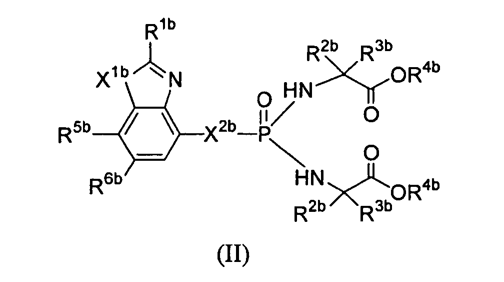 wherein X^{1b} represents an oxygen atom or a sulfur atom; X^{2b} represents a C₁₋₄ alkylene group, a C₁₋₄ oxyalkylene group (with the proviso that the phosphorous atom is attached to a carbon atom), or a C₁₋₄ thioalkylene group (with the proviso that the phosphorous atom is attached to a carbon atom); R^{1b} represents a hydrogen atom, a halogen atom, a C₁₋₆ alkyl group or an amino group (said amino group may optionally be substituted with one or two C₁₋₆ alkyl groups); R^{2b} and R^{3b} are the same or different, and each represents a hydrogen atom or a C₁₋₄ alkyl group; R^{4b} represents a C₁₋₄ alkyl group; and R^{5b} and R^{6b} are the same or different, and each represents a hydrogen atom, a halogen atom, a C₁₋₆ alkyl group, a C₁₋₆ alkoxy group, or an amino group (said amino group may optionally be substituted with one or two C₁₋₆ alkyl groups).

46. A method for the prevention or treatment of diseases according to claims 38 to 42 wherein wherein the FBPase inhibitor is 2-amino-5-isobutyl-4-{2-[5-(N,N'-bis((S)-1-ethoxycarbonyl)ethyl)phosphonamido]furanyl}thiazole, 2-amino-5-isobutyl-4-{2-[5-(O-(2-bis(N-(1-methyl-1-ethoxycarbonyl)ethyl)phosphonamido)furanyl)-thiazole, 2-amino-5-propylthio-4-{2-[5-(N,N'-(1-(S)ethoxycarbonyl)ethyl)-phosphonamido]furanyl}thiazole, 2-amino-5-propylthio-4-{2-[5-(N,N'-(1-methyl-ethoxycarbonyl)ethyl)phosphonamido]furanyl}thiazole, or a pharmacologically acceptable salt thereof.

47. An administration of an FBPase inhibitor for the prevention of diabetes mellitus.

48. An administration of an FBPase inhibitor for the treatment of impaired glucose tolerance.

49. An administration of an FBPase inhibitor for the prevention of diseases related to impaired glucose tolerance.

50. An administration according to claim 49 wherein the diseases related to impaired glucose tolerance are macroangiopathy or arteriosclerosis.

51. An administration according to claims 47 to 50 wherein the FBPase inhibitor is a compound of the following formula (I) or a pharmacologically acceptable salt thereof: 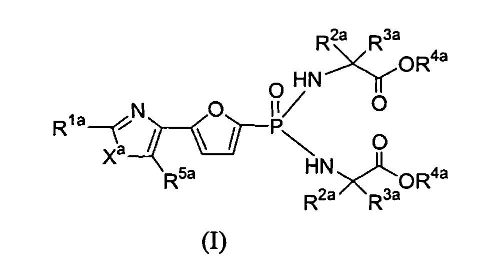 wherein X^{a} represents a nitrogen atom, an oxygen atom or a sulfur atom; R^{1a} represents a hydrogen atom, a halogen atom, a C₁₋₆ alkyl group, or an amino group (said amino group may optionally be substituted with one or two C₁₋₆ alkyl groups); R^{2a} and R^{3a} are the same or different, and each represents a hydrogen atom or a C₁₋₄ alkyl group; R^{4a} represents a C₁₋₄ alkyl group; and R^{5a} represents a hydrogen atom, a C₁₋₆ alkyl group or a C₁₋₆ alkylthio group.

52. An administration according to claims 47 to 50 wherein the FBPase inhibitor is a compound of the following formula (Ia) or a pharmacologically acceptable salt thereof: 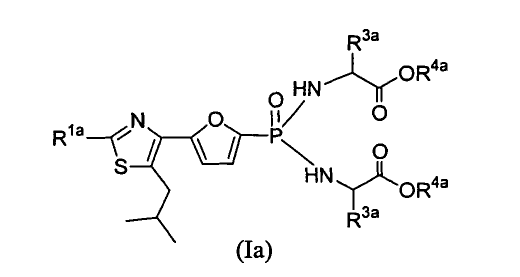 wherein R^{1a} represents an amino group (said amino group may optionally be substituted with one or two C₁₋₆ alkyl groups); R^{3a} represents a hydrogen atom or a C₁₋₄ alkyl group; and R^{4a} represents a C₁₋₄ alkyl group.

53. An administration according to claims 47 to 50 wherein the FBPase inhibitor is a compound of the following formula (II) or a pharmacologically acceptable salt thereof: 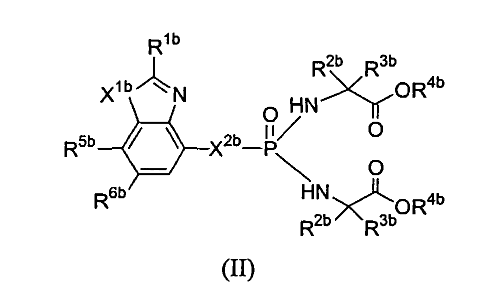 wherein X^{1b} represents an oxygen atom or a sulfur atom; X^{2b} represents a C₁₋₄ alkylene group, a C₁₋₄ oxyalkylene group (with the proviso that the phosphorous atom is attached to a carbon atom), or a C₁₋₄ thioalkylene group (with the proviso that the phosphorous atom is attached to a carbon atom); R^{1b} represents a hydrogen atom, a halogen atom, a C₁₋₆ alkyl group or an amino group (said amino group may optionally be substituted with one or two C₁₋₆ alkyl groups); R^{2b} and R^{3b} are the same or different, and each represents a hydrogen atom or a C₁₋₄ alkyl group; R^{4b} represents a C₁₋₄ alkyl group; and R^{5b} and R^{6b} are the same or different, and each represents a hydrogen atom, a halogen atom, a C₁₋₆ alkyl group, a C₁₋₆ alkoxy group, or an amino group (said amino group may optionally be substituted with one or two C₁₋₆ alkyl groups).

54. An administration according to claims 47 to 50 wherein the FBPase inhibitor is 2-amino-5-isobutyl-4-{2-[5-(N,N'-bis((S)-1-ethoxycarbonyl)ethyl)phosphonamido]-furanyl}thiazole, 2-amino-5-isobutyl-4-{2-[5-(O-(2-bis(N-(1-methyl-1-ethoxycarbonyl)ethyl)phosphonamido)furanyl)thiazole, 2-amino-5-propylthio-4-{2-[5-(N,N'-(1-(S)ethoxycarbonyl)ethyl)phosphonamido]furanyl}thiazole, 2-amino-5-propylthio-4-{2-[5-(N,N'-(1-methyl-ethoxycarbonyl)ethyl)phosphonamido]furanyl}-thiazole, or a pharmacologically acceptable salt thereof.
